# EUROPEAN PATENT APPLICATION

(11) **EP 3 542 782 A1**
(43) Date of publication of application: **25.09.2019**
(21) Application number: 18163206.8
(22) Date of filing: 21.03.2018
(51) Int. Cl.: A61K 8/29, A61Q 17/04, A61K 8/02

(54) **TITANIUM DIOXIDE**

(71) Applicant: Venator Germany GmbH, 47198 Duisburg (DE); Venator P&A Finland Oy, 28840 Pori (FI); Venator Materials UK Limited, Stockton-on-Tees, Durham TS22 5FD (GB)
(72) Inventor: JOHN, Stephan, 47198 Duisburg (DE); LATVA-NIRVA, Esa, 28430 Pori (FI); ROBB, John, Stockton-on-Tees, Durham TS18 5EG (GB)
(74) Representative: Barker Brettell LLP

(57) **Abstract**

A cosmetic composition is provided that comprises from 0.1 to 20wt% of an organic cosmetic active ingredient that has ligand characteristics, such as avobenzone; from 0.1 to 30wt% of titanium dioxide particulate material, wherein the titanium dioxide is in the rutile form and has a geometric weight mean crystal size of from 0.35µm to 5µm, and wherein the titanium dioxide particles are provided with a silica coating; and a cosmetically acceptable carrier. The cosmetic composition can be used as a broad spectrum sunscreen and has good stability, with reduced discoloration.

## Description

The present invention relates to cosmetic compositions, including, but not limited to, sunscreen formulations.

### Background to the Invention

The damaging effects of sunlight on skin are well documented. Significant damage can be done to the skin just by routine day-to-day activities in sunlight. In addition, sunbathing can clearly also cause skin damage. The major short term hazard of prolonged exposure to sunlight is erythema, i.e. sunburn. In addition to the short term hazard there are long term hazards, such as malignant changes in the skin surface.

Ultraviolet (UV) radiation covers three wavelength regions: UV-A (320 nm-400 nm), UV-B (280 nm-320 nm) and UV-C (100 nm-280 nm).

Numerous epidemiologic studies demonstrate a strong relationship between UV exposure, especially UV-B exposure, and human skin cancer. Another long term hazard of ultraviolet radiation in both the UV-A and UV-B regions is premature aging of the skin. This condition is characterized by wrinkling and pigment changes of the skin, along with other physical changes such as cracking, telangiectasia, solar dermatoses, ecchymosis, and loss of elasticity.

The sun protection product market has grown considerably over the years and many new products are introduced each year. What used to be looked upon as a seasonal business is no longer seen as such. Sunscreen agents such as UV-A and UV-B filters are now included in a diversity of personal care products, particularly cosmetic type products which are worn on a daily basis.

Scientific studies have shown that IR radiation can also cause damage to skin cells. Although this type of radiation is less energetic than UV radiation, it will cause relaxation of the skin and formation of fine lines and wrinkles. Therefore solar IR is expected to have significant biological effects on human skin.

Infrared (IR) radiation covers three wavelength regions: IR-A (760-1400 nm), IR-B (1400-3000 nm), and IR-C (3000 nm-1 mm). Half of the solar energy reaching the earth's surface is in the overall IR range, of from 760nm to 1mm, with 2% being ultraviolet (UV) and 48% being visible.

IR-A can penetrate epidermal and dermal layers and reach subcutaneous tissues without increasing the skin temperature significantly, whereas IR-B and IR-C are absorbed mostly in the epidermal layers and increase skin temperature significantly. Chronic heat exposure of human skin may cause alterations to the skin. For example, the skin condition erythema ab igne (EAI) is caused by chronic exposure to infrared radiation in the form of heat.

IR and heat exposure can induce cutaneous angiogenesis and inflammatory cellular infiltration, disrupt the dermal extracellular matrix by inducing matrix metalloproteinases, and alter dermal structural proteins leading to premature skin aging. IR exposure is also linked to induced reactive oxygen species that are suspected to cause skin cancer.

The cosmetic industry offers products that claim to reduce skin damage from the harmful results of the IR-induced effects by including certain antioxidants. However, whilst these products may serve to limit the impact of reactive oxygen species, cosmetic ingredients that prevent or reduce the formation of reactive oxygen species by IR radiation remain uncommon.

Exposure to visible light may be considered unavoidable. However, visible light has also been observed to cause undesirable changes within the skin.

Therefore all solar radiation can be harmful to the skin and protection against a broad spectrum of wavelengths is desirable.

EP1580166 describes titanium dioxide particles which are stated to have highly selective shielding of thermal infrared radiation. These particles have a primary particle size (i.e. a crystal size) between 0.5 and 2.0µm. They are produced from hydrated TiO₂ which is blended with an aluminum compound, a potassium compound, and a zinc compound, then dried, and calcined at a temperature between 900°C and 1,100°C. The TiO₂ particles thus produced contain at least 0.05 to 0.4% by weight of Al₂O₃ and 0.05 to 0.5% by weight of ZnO. They are described as being able to be incorporated into paints, printing inks or plastic molding compounds for shielding thermal IR radiation, and for incorporation into cosmetic preparations.

EP2285912 describes a coloured composition comprising: titanium dioxide particulate material and non-white colorant, dispersed within a vehicle. The TiO₂ particulate material has an average crystal size of greater than 0.40 µm and a particle size distribution such that 30% or more of the particles are less than 1 µm. The TiO₂ particulate material is coated with two or more oxide materials, wherein one of these oxide materials is a dense silica material.

US2012/015015 provides a composite powder comprising infrared-ray blocking particles and ultraviolet-ray blocking particles coated onto the surface of the infrared-ray blocking particles. The diameter of the infrared-ray blocking particle may be within the range of 0.38 to 1.5µm. The diameter of the ultraviolet-ray blocking particles may be within the range of 8 to 150nm. The composite powder is described for use in cosmetics.

In general, titanium dioxide is known as a valuable component of cosmetic formulations. However, the use of TiO₂ particles is limited by compatibility issues.

Avobenzone, also known as 1-(4-methoxyphenyl)-3-(4-tert-butylphenyl) propane-1,3-dione, is an absorber of UV radiation. It is commonly used in sunscreen formulations due to its ability to absorb the full spectrum of UV-A radiation, with an absorption maximum of 357 nm. Avobenzone was approved for cosmetic use in Europe in 1978, in the United States in 1988.

Avobenzone should be complementary to mineral sunscreens, which tend to be more effective in the UV-B region. However, mineral products including titanium dioxide and zinc oxide have been found to react with avobenzone on storage. This reaction produces discoloration which can lead to precipitation or crystallisation within the formulation. It is also the case that the discoloration is indicative of a loss of sun-blocking efficacy.

It is a known problem that avobenzone undergoes keto-enol isomerization. An enolate anion forms that can chelate with cations, e.g. iron, aluminium or zinc cations, producing a coloured, water-insoluble complex. Additionally, the complex can chemically destabilize the avobenzone molecule so that it is subject to cleavage mechanisms.

Cosmetics frequently utilize iron-containing compounds as colorants, astringents, and skin conditioning agents. Therefore these chelation problems are commonly encountered.

In addition to these chelation problems, many UV absorbers, including avobenzone, exhibit photolability, in which absorbed energy causes photodegradation and/or photoreactivity, and thus reduces its efficacy.

WO 2012/078961 recognises this stability problem for avobenzone, and provides certain chelating polymers for use with avobenzone, which are described as reducing or preventing formation of the avobenzone-iron chelate.

Dihydroxyacetone (DHA) is well known as a self-tanning agent but represents a further example of a product that can have degradation and stability issues. In the presence of some forms of titanium dioxide it can decompose, with a loss of efficacy as well as a discoloration from yellow to brown.

Antioxidants including ascorbyl palmitate and propyl gallate can also discolour in the presence of titanium dioxide, whilst other antioxidants can suffer a loss of efficacy without discoloration in the presence of titanium dioxide, e.g. di-alpha- tocopherol (vitamin E), di-alpha-tocopheryl acetate and vitamin A-palmitate.

Meanwhile, polyacrylates used as synthetic emulsifiers in cosmetic emulsions can be affected by the presence of titanium dioxide, with a loss of efficacy leading to the emulsion destabilising.

Therefore it remains a problem that TiO₂ products are known to interact on storage with certain common cosmetic components with detrimental effects, such as loss of efficacy and/or producing undesirable (yellow, brown or red) discolorations. In fact, discoloration can be an indication of loss of efficacy.

Therefore there remains a need to stabilize avobenzone and other cosmetic components in the presence of TiO₂.

### Summary of the Invention

The invention provides, in a first aspect, a cosmetic composition that comprises:
- from 0.1 to 20wt% of an organic cosmetic active ingredient that has ligand characteristics;
- from 0.1 to 30wt% of titanium dioxide particulate material, wherein the titanium dioxide is in the rutile form and has a geometric weight mean crystal size of from 0.35µm to 5µm, and wherein the titanium dioxide particles are provided with a silica coating; and
- a cosmetically acceptable carrier.

Unexpectedly, the cosmetic composition of the first aspect has reduced detrimental effects (e.g. discoloration and/or loss of efficacy) as compared to what would be expected for a composition that comprises an organic cosmetic active ingredient that has ligand characteristics (such as avobenzone) together with a mineral product such as titanium dioxide (e.g. with reference to ultrafine or pigmentary rutile TiO₂ with a geometric weight mean crystal size of up to 0.3µm).

In particular, the cosmetic composition of the first aspect has less discolouration than would be expected for a composition comprising an organic cosmetic active ingredient that has ligand characteristics, such as avobenzone, together with a mineral product such as titanium dioxide (e.g. with reference to ultrafine or pigmentary rutile TiO₂ with a geometric weight mean crystal size of up to 0.3µm). Where reference is made to discoloration this means a change in colour. A change in colour can be measured by measuring ΔE*, which is the measured distance in perceptual color space, using a colorimeter, such as a Konica Minolta CR-410 Colorimeter. Differences above 0.2, such as 0.5 or more or 1.0 or more, can be considered a discoloration.

The use of the specific claimed type of titanium dioxide, which is in the rutile form and has a geometric weight mean crystal size of from 0.35µm to 5µm, and wherein the titanium dioxide particles are provided with a silica coating, has been surprisingly found to prevent or reduce detrimental effects (e.g. discoloration and/or loss of efficacy) for organic cosmetic active ingredients that have ligand characteristics. In particular, it has been shown to prevent or reduce the discoloration of compositions that contain active cosmetic components such as avobenzone, ascorbyl palmitate and propyl gallate.

The invention therefore also provides, in a second aspect, the use of titanium dioxide particulate material, wherein the titanium dioxide is in the rutile form and has a geometric weight mean crystal size of from 0.35µm to 5µm, and wherein the titanium dioxide particles are provided with a silica coating, to prevent or reduce detrimental effects (e.g. discoloration and/or loss of efficacy) in relation to an organic cosmetic active ingredient that has ligand characteristics.

In particular, by using this specific titanium dioxide particulate material in combination with the organic cosmetic active ingredient that has ligand characteristics in a cosmetic composition, there is a prevention or reduction of detrimental effects that are associated with using the organic cosmetic active ingredient that has ligand characteristics in a cosmetic composition together with conventional titanium dioxide particulate material. This may in particular be with reference to ultrafine or pigmentary rutile TiO₂ with a geometric weight mean crystal size of up to 0.3µm.

In one embodiment, the specific titanium dioxide particulate material according to the claimed invention is used in place of the conventional titanium dioxide particulate material, e.g. the ultrafine or pigmentary rutile TiO₂ with a geometric weight mean crystal size of up to 0.3µm. In another embodiment, the specific titanium dioxide particulate material according to the claimed invention is used in addition to the conventional titanium dioxide particulate material, e.g. the ultrafine or pigmentary rutile TiO₂ with a geometric weight mean crystal size of up to 0.3µm.

In one embodiment there is a reduction in discoloration. The reduction of discoloration for the organic cosmetic active ingredient that has ligand characteristics may be seen by reference to the ΔE* value when using the defined titanium dioxide particulate material as compared to using a reference which is ultrafine or pigmentary rutile TiO₂ with a geometric weight mean crystal size of up to 0.3µm. The ΔE* value as determined after 7 days storage in dark conditions may be reduced by 10% or more, such as 20% or more, or 30% or more, or 40% or more. In some cases it may be reduced by 50% or more, such as 60% or more, or 70% or more, or 80% or more.

Furthermore, the titanium dioxide material as used in the cosmetic composition of the first aspect is beneficial in that it not only has IR protective effects but also contributes a UV protective effect. Therefore the composition can be used as a broad spectrum sunscreen. The composition can be used to protect the skin not only against UV and visible radiation, and induced harms from those wavelengths, but also against infrared radiation and induced negative effects, and against combinations of wavelengths.

The invention therefore also provides, in a third aspect, the use of the cosmetic composition as defined in the first aspect in a method of preventing or reducing damage to human skin from the harmful effects of solar radiation. In this regard, the cosmetic composition can be applied to the human skin prior to exposure to the sun. The composition may in one embodiment provide protection against both UV and IR radiation.

The titanium dioxide material as used in the cosmetic composition of the first aspect is also beneficial in that it has a colour that is similar to natural flesh tone. This contrasts with smaller crystal size titania that is blue in tone. Therefore the cosmetic composition of the first aspect can usefully be used in cosmetic products such as foundation and face powder.

The invention further provides, in a fourth aspect, a cosmetic composition that comprises:
- from 0.1 to 30wt% of titanium dioxide particulate material, wherein the titanium dioxide is in the rutile form and has a geometric weight mean crystal size of from 0.35µm to 5µm, and wherein the titanium dioxide particles are provided with a silica coating;
- from 0.1 to 30wt% of titanium dioxide particulate material, wherein the titanium dioxide is in the rutile form and has a geometric weight mean crystal size of up to 0.2µm and
- a cosmetically acceptable carrier.

It has been found that the use of (a) the specific claimed type of large crystal titanium dioxide - which is in the rutile form and has a geometric weight mean crystal size of from 0.35µm to 5µm, and wherein the titanium dioxide particles are provided with a silica coating - in combination with (b) small crystal titanium dioxide - which is in the rutile form and has a geometric weight mean crystal size of up to 0.2µm - leads to a synergistic effect. In this regard, the SPF values for compositions that include the specific claimed type of large crystal titanium dioxide together with small crystal titanium dioxide have been unexpectedly found to be greater than the additive effect of the two materials.

It would not be predicted that the use of two types of titanium dioxide in combination could lead to a much greater SPF value than the sum of the SPF values for the two types of titanium dioxide when used alone.

In one embodiment, the invention provides a cosmetic composition that comprises:
- from 0.1 to 20wt% of an organic cosmetic active ingredient that has ligand characteristics;
- from 0.1 to 30wt% of titanium dioxide particulate material, wherein the titanium dioxide is in the rutile form and has a geometric weight mean crystal size of from 0.35µm to 5µm, and wherein the titanium dioxide particles are provided with a silica coating;
- from 0.1 to 30wt% of titanium dioxide particulate material, wherein the titanium dioxide is in the rutile form and has a geometric weight mean crystal size of up to 0.2µm and
- a cosmetically acceptable carrier.

This composition has all of the above noted benefits and therefore is particularly advantageous.

The compositions of the invention can comprise, consist essentially of, or consist solely of, the essential ingredients as well as any or all optional ingredients described herein. Whenever amounts are given, these are by weight unless stated otherwise or unless the context makes it clear that it is otherwise.

### Brief Description of the Drawings

The accompanying drawings, which are incorporated in and form part of the specification, illustrate embodiments of the present invention and serve to illustrate rather than limit the invention.

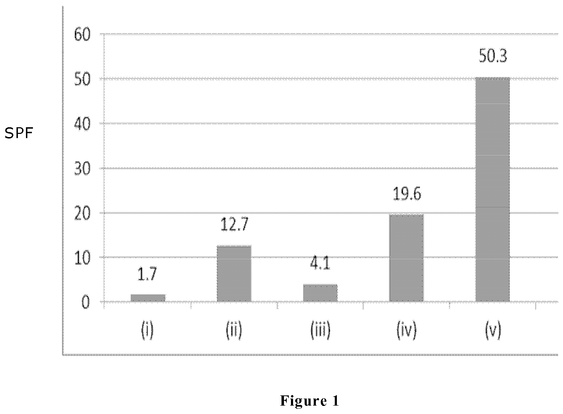

**Figure 1** is a bar chart showing the SPF values for titanium oxide containing formulations prepared in Example 3.

### Detailed Description of the Invention

### "Large crystal" TiO₂ material

The cosmetic compositions of the invention comprise titanium dioxide in the rutile form and having a mean crystal size of from 0.35µm to 5µm. Thus this is a large crystal size pigment. It is known from, for example, WO2009/136141 and WO2016/128723, that such material is useful for scattering the near infrared part of the electromagnetic spectrum.

In the present invention, it has been determined that this specific subset of titania materials not only has IR protective effects but also contributes a UV protective effect.

Furthermore, this specific subset of titania materials acts to prevent or reduce detrimental effects, such as discoloration and/or loss of efficacy and/or emulsion destabilisation, in relation to cosmetic active ingredients that have ligand characteristics. In particular, this specific subset of titania materials acts to prevent or reduce the discoloration of cosmetic active ingredients that have ligand characteristics, such as avobenzone, ascorbyl palmitate and propyl gallate.

In addition, it has been determined that this specific subset of titania materials have a synergistic effect when used in combination with small crystal titanium dioxide, such as ultrafine or pigmentary titania. The overall SPF values for the combination are greater than the sum of the SPF values for the titania materials when used individually.

In the present invention the upper limit on crystal size for the large crystal size titania is determined by the need for a cosmetic composition to not look or feel gritty. If the mean crystal size is above 5µm, the composition will not meet these characteristics.

In one embodiment the titanium dioxide has a mean crystal size of up to 4µm. For example, the titanium dioxide may have a mean crystal size of up to 3.8µm, e.g. up to 3.6µm. It may be that the mean crystal size is up to 3.4 µm, e.g. up to 3.2 µm, or up to 3µm. It may be that the mean crystal size is up to 2.8 µm, or up to 2.6 µm e.g. up to 2.4 µm, or up to 2.2µm.

In one embodiment the titanium dioxide has a mean crystal size of up to 2.0 µm. For example, the titanium dioxide may have a mean crystal size of up to 1.9µm, e.g. up to 1.8µm. It may be that the mean crystal size is up to 1.7 µm, e.g. up to 1.6 µm. In one embodiment, the titanium dioxide has a mean crystal size from 0.35 to 2µm or from 0.40 to 2µm.

In one embodiment the titanium dioxide has a mean crystal size of up to 1.5 µm. This maximum limit on the size is preferred from the perspective of ease of manufacture. Titanium dioxide with crystal sizes above 1.5 µm can be made, but may require higher temperatures than those used in conventional processing.

The beneficial effects of the titanium dioxide are seen at mean crystal sizes of 0.35µm and above.

In one embodiment, therefore, the titanium dioxide has a mean crystal size from 0.35 to 1.5µm, e.g. from 0.35 to 1.4µm, or from 0.35 to 1.3µm, or from 0.35 to 1.2µm.

In one embodiment, the titanium dioxide has a mean crystal size from 0.40 to 1.5µm, e.g. from 0.40 to 1.4µm, or from 0.40 to 1.3µm, or from 0.4 to 1.2µm, or from 0.4 to 1.1µm.

It may be that the titanium dioxide has a mean crystal size from 0.35 to 0.7µm, e.g. from 0.4 to 0.7µm. The compositions where the titania crystal size is in the range of 0.4 to 0.7 microns are more opaque than the compositions where the titania crystal size is in the range of above 0.7 microns. It will be appreciated that for some cosmetic formulations, opacity is desirable, e.g. when the formulation will cover undesired features on the skin (such as pigmentation or discoloration, marks or scars, and blemishes).

It may be that the titanium dioxide has a mean crystal size from 0.7 to 1.5µm, e.g. from 0.7 to 1.2µm or from 0.7 to 1.0µm. It will be appreciated that for some cosmetic formulations, translucency is desirable, e.g. sunscreens that are translucent can be popular.

Mean crystal size may be determined by transmission electron microscopy on a rubbed out sample with image analysis of the resulting photograph (e.g. using a Quantimet 570 Image Analyser). This may be validated by reference to the latex NANOSPHERE ™ size standard 3200 from NIST with a certified size of 199+/-6nm. The crystal size may be determined for uncoated TiO₂ (although the skilled reader will appreciate that the thickness of the coating is of a magnitude of just a few nm, such that in practice there is no measurable difference in the crystal size for the corresponding coated products, when taking into account a suitable degree of accuracy for the claimed dimensions).

Conventional rutile TiO₂ has a mean crystal size of from 0.17 to 0.29 µm, whilst conventional anatase TiO₂ has a mean crystal size of from 0.10 to 0.25 µm.

Crystal size is distinct from particle size. The particle size depends on the effectiveness of the dispersion of the pigment in the system within which it is used. Particle size is determined by factors such as crystal size and milling techniques, e.g. dry, wet or incorporative milling. The particle size of conventional rutile TiO₂ is from 0.25 to 0.40 µm, whilst conventional anatase TiO₂ has a particle size of from 0.20 to 0.40 µm. Larger particle sizes can result if the techniques used are such that crystals "clump" together.

In the present invention, the titanium dioxide may suitable have a mean particle size, as determined by X-ray sedimentation, of greater than 0.4µm. For example, the mean particle size may be greater than 0.4µm and up to 5µm.

In one embodiment, the titanium dioxide used has a particle size distribution such that 30% or more of the particles are less than 1.5 micron. This may be measured by using a Brookhaven X-ray disk centrifuge. In another embodiment, the titanium dioxide used has a particle size distribution such that 30% or more of the particles are less than 1 micron.

The titanium dioxide can be prepared by any known method. For example, the so-called "sulphate" route or the so-called "chloride" route may be used, which are the two routes in wide commercial use. Equally, the fluoride process, hydrothermal processes, aerosol processes or leaching processes may be used to prepare the titanium dioxide.

### a) Nature of the TiO₂ before coating

It will be appreciated by the skilled reader that the following discussions of the nature and characteristics of the titanium dioxide relate to its form before the coating is applied.

The titanium dioxide used in the present invention is in the rutile crystal form. In this regard, the titanium dioxide is required to be 50% or more by weight rutile, such as 60% or more, e.g. 70% or more, preferably 80% or more, more preferably 90% or more, most preferably 95% or more, such as 99% or more, for example 99.5% or more.

The titanium dioxide may be white or translucent or may be coloured. In one embodiment, it may be substantially white; for example it may have a lightness value L* (CIE L*a*b* colour space) of greater than 95, with a value of a* of less than 5 and a value of b* of less than 5.

The titanium dioxide may include impurities provided that these are cosmetically acceptable. It may, for example, include impurities up to a level of 10wt% or less; such as 8wt% or less, e.g. 5wt% or less or 2wt% or less. These impurities result from incomplete purification and may, for example, be iron, silica, niobia or other impurities typically present in titanium dioxide-bearing feedstocks. In one embodiment the titanium dioxide may include impurities up to a level of 0.5wt% or less, such as 0.1wt% or less, e.g. 0.01wt% or less; these impurities may, for example, be iron, phosphorous, niobia or other impurities typically present in titanium dioxide bearing feedstocks.

Preferably the titanium dioxide has a TiO₂ content of 90wt% or higher, such as 92wt% or higher, for example 93wt% or higher. More preferably the titanium dioxide has a TiO₂ content of 95wt% or higher, such as 99wt% or higher, for example 99.5wt% or higher.

The titanium dioxide used in the present invention may optionally be doped, but in a preferred embodiment it is not doped. In one embodiment, the particulate material is or comprises a doped titanium dioxide, that is to say an inorganic material containing TiO₂. The doped titanium dioxide may have a TiO₂ content of 50wt% or more, preferably 60wt% or more, such as 65wt% or more, or 70wt% or more. Preferably the doped titanium dioxide may have a TiO₂ content of 80wt% or more, preferably 90wt% or more.

The doped titanium dioxide possesses the rutile crystal structure. As the skilled person will appreciate, this does not necessarily mean that the doped titanium dioxide is rutile but can be material which is iso-structural with rutile.

The doped titanium dioxide may, for example, be doped with dopants such as calcium, magnesium, sodium, phosphorus, and caesium.

The doped titanium dioxide may include impurities, e.g. up to a level of 10wt% or less, such as 8wt% or less, e.g. 5wt% or less. These impurities result from incomplete purification and may, for example, be iron, silica, niobia or other impurities typically present in titanium dioxide bearing feedstocks.

The titanium oxide may have a lattice that is doped with an impurity which acts as a recombination centre for holes and electrons. For example, Cr, Mn and V can all be used as dopants to promote recombination. These impurities tend to be added in the form of a salt before calcination, by addition of the salt to the precipitated slurry/pulp. Alternatively the impurities can be allowed to come through from the titanium ore, in controlled quantities. The amounts of dopant used are typically from 2 to 10ppm because the durability benefit has to be balanced against colour deterioration.

### b) Nature of the coating

The titanium dioxide is coated with silica. In this regard, a dense or non-dense silica coating may be used.

When reference is made to the titanium dioxide being coated with silica, this refers the titanium dioxide particles being coated with a coating layer that comprises silica, e.g. 50%w/w or more, such as 75%w/w or more or 90%w/w or more, of the material for the coating layer may be silica.

In one embodiment, the coating layer consists essentially of silica. In one such embodiment, the coating layer is silica.

The silica coating encapsulates the titanium dioxide particles and provides an impervious coating.

A coating agent can be used to apply the coating layer. This may, for example, be SiO₂, sodium silicate, potassium silicate, or mixtures thereof. Silicic acid may also be mentioned.

In one embodiment the coating agent used to apply the coating layer comprises silicon dioxide applied in a dense form. In one such embodiment, the coating comprises a dense silica coating of the type as described in US 2,885,366.

A dense silica coating may be applied by following a recipe along the following lines:

| | **gpl TiO2** | **Temp °C** | | | |
|---|---|---|---|---|---|
| | 350 | 90 | | | |

| Instruction | | Reagent | | Addition time (minutes) | Mixing time minutes |
|---|---|---|---|---|---|
| | | | | | |
| ADD x % SiO₂ | | Na₂SiO₃ | | 45 | 30 |
| | | | | | |
| ADD H₂SO₄ to pH 7.5 | | H₂SO₄ | | 60 | 30 |

Surface treatments of inorganic particles with oxide materials are well known in the art. Therefore, any suitable technique can be used in the step of applying the silica coating onto the particles.

In one embodiment of the present invention, the amount of the silica coating on the titanium dioxide particles is from 0.1 to 10% w/w, when considering the total weight of the silica with respect to the total weight of the particulate titanium dioxide, e.g. from 0.1 to 9% w/w, or from 0.1 to 8% w/w, or from 0.1 to 7% w/w.

In one embodiment of the present invention, the amount of the silica coating on the titanium dioxide particles is from 0.1 to 6% w/w, when considering the total weight of the silica with respect to the total weight of the particulate titanium dioxide, e.g. from 0.1 to 5% w/w or from 0.1 to 4% w/w.

In one embodiment of the present invention, the amount of the silica coating on the titanium dioxide particles is from 0.15 to 6% w/w, when considering the total weight of the silica with respect to the total weight of the particulate titanium dioxide, e.g. from 0.15 to 5% w/w or from 0.15 to 4% w/w.

In one embodiment of the present invention, the amount of the silica coating on the titanium dioxide particles is from 0.25 to 6% w/w, when considering the total weight of the silica with respect to the total weight of the particulate titanium dioxide, e.g. from 0.25 to 5% w/w or from 0.25 to 4% w/w.

It may be that the amount of the silica coating on the titanium dioxide particles is from 0.5 to 6% w/w, when considering the total weight of the silica with respect to the total weight of the particulate titanium dioxide; such as from 0.5 to 5.5% w/w, or from 0.5 to 5% w/w, or from 0.5 to 4.5% w/w, or from 0.5 to 4% w/w, or from 0.5 to 3.5% w/w.

It may be that the amount of the silica coating on the titanium dioxide particles is from 1 to 6% w/w, when considering the total weight of the silica with respect to the total weight of the particulate titanium dioxide; such as from 1 to 5.5% w/w, or from 1 to 5% w/w, or from 1 to 4.5% w/w, or from 1 to 4% w/w, or from 1 to 3.5% w/w. Preferably, it may be from 1 to 3% w/w.

When reference is made to an addition level of coating on the titanium dioxide particles, this is given as a w/w amount, i.e. the total weight amount of coating material that is added with respect to the total weight amount of titanium dioxide particles treated. Thus, for example, when considering a silica coating, it may be stated that "the addition level of the SiO₂ was 1.5% w/w on to the TiO₂".

The coating material may be applied to titanium dioxide particles in the form of a dispersion. This may be by adding the coating material to the dispersion or by adding the dispersion to the coating material. Preferably, mixing of the coating material and dispersion is carried out using conventional mixing equipment as known in the art.

Mixing may be carried out for any suitable length of time, e.g. 1 minute or more, 2 minutes or more, 3 minutes or more, 4 minutes or more, or 5 minutes or more. It may be that mixing is carried out for no more than 3 hours, e.g. no more than 2 hours, such as 1 hour or less. In one embodiment the mixing is carried out for from 5 minutes to 1 hour, such as from 10 minutes to 45 minutes, e.g. from 20 minutes to 40 minutes.

It is to be noted that the coating does not immediately react when added. Instead, as the skilled person will appreciate, the coating reacts/precipitates in response to a subsequent pH change. In the case of silica, the application of an integral dense coating is dependent on the rate of pH change once the reagents are in the tank. This rate of pH change is typically from minus 1 to minus 2 units per hour, e.g. about minus 1.5 units in 1 hour.

In one embodiment, a coating may be applied as follows: an aqueous dispersion comprising particles of titanium dioxide is introduced into a tank for stirring. The temperature of the dispersion is then adjusted (e.g. to 75 to 90°C) and its pH is adjusted (e.g. to about 10.5). A coating material is then introduced into the stirred tank in an amount sufficient to produce the desired coating. For example, to produce a 1% by weight dense silica coating, 1% silica (%wt/wt on titanium dioxide) is added to the stirred tank over a 30 minute period and is then mixed for 30 minutes; whilst to produce a 3% by weight dense silica coating, 3% silica (%wt/wt on titanium dioxide) is added in the same manner. In one embodiment, silica may be added to the stirred tank in the form of sodium silicate as coating material. To precipitate the dense silica coating onto the particles, the pH is adjusted, e.g. by adding sulphuric acid to the stirred tank. In one particular embodiment, sulphuric acid is added over a 60 minute period to bring the pH to about 8.

The skilled reader will of course appreciate that this method can readily be modified to add different amounts of coating, as desired and within the ranges of the invention. The coating of titania by silica can readily be put into practice by the skilled person.

The titanium dioxide may optionally have one or more further coating layers. These further coating layers may be above or below the silica coating. In other words, it may be that the further coating layer is adjacent the titanium dioxide particle surface, and the silica coating layer is then on top of the further coating layer, or it may be that the silica coating layer is adjacent the titanium dioxide particle surface, and the further coating layer is then on top of the silica coating layer.

In one embodiment, there is no further coating layer, i.e. the only coating is silica.

In one embodiment, there is a further coating layer and this comprises alumina. In another embodiment, there is a further coating layer and this does not comprise alumina. It can be preferred that there is no alumina present because this material is often considered undesirable within the cosmetic industry. Alumina has also been implicated in avobenzone discoloration. Another drawback of alumina being present is that this leads to strong opacity in the visible light area, which is not always desired.

In one embodiment, there is a further coating layer and this comprises one or more material selected from inorganic oxides and phosphates. For example, it may comprise one or more inorganic oxide independently selected from an oxide of Ti, Zr, Zn, P, Sn and Ce and/or one or more inorganic phosphate independently selected from a phosphate of Al, Ti, Zr, and Sn.

It may suitably be that the material for the further coating layer is one or more inorganic oxide independently selected from ZrO₂, CeO₂, and P₂O₅ and/or one or more inorganic phosphate independently selected from AlPO₄ and ZrPO₄.

It will be appreciated that in some embodiments the material for the further coating layer is only one inorganic oxide. For example, it may be just ZrO₂, or just CeO₂, or just P₂O₅. In other embodiments, the material for the first layer is two inorganic oxides. For example, it may be SiO₂ with ZrO₂, or it may be SiO₂ with CeO₂ or it may be SiO₂ with P₂O₅ or it may be ZrO₂ with CeO₂ or it may be ZrO₂ with P₂O₅ or it may be CeO₂ with P₂O₅

In another embodiment the material for the first layer is only one inorganic phosphate. For example, it may be just AlPO₄ (which, as the skilled person will appreciate, is isostructural with silica and can form a useful dense coating) or it may be just ZrPO₄.

Where there is a further layer, the amount of the further coating material on the titanium dioxide particles may be from 0.1 to 6% w/w, when considering the total weight of the coating material with respect to the total weight of the particulate titanium dioxide, e.g. from 0.1 to 5% w/w or from 0.1 to 4% w/w, such as from 0.5 to 4% w/w, or from 0.5 to 3.5% w/w, or from 0.5 to 3% w/w, or from 0.5 to 2.5% w/w, or from 0.5 to 2% w/w.

In one embodiment, the total amount of coating (silica coating layer plus any further coating layer) is from 0.15 to 10% w/w, e.g. from 0.25 to 6% w/w, such as from 0.5 to 5% w/w or from 1 to 4% w/w, when considering the total weight of the coating material with respect to the total weight of the particulate titanium dioxide.

In one embodiment, the particles are further treated with coagulant or a dispersive agent. This is suitably carried out after the coating steps. The particulate inorganic material may be subjected to a further inorganic surface treatment and/or organic surface treatment. The treatment may, for example, be at a level of from 0.1 to 5wt%, e.g. from 0.25 to 4wt%, or from 0.5 to 2wt%.

An organic surface treatment, such as with polyol, amine (e.g. an alkanolamine) or silicone derivatives, may be used in one embodiment. This may, in particular, improve dispersability. Examples of organic compounds that may be used are trimethylolpropane, pentaerythritol, triethanolamine, n-octyl phosphonic acid and trimethylolethane.

In one embodiment the particles are treated with a silicone (or polysiloxane), e.g. polydimethylsiloxane. The treatment may be at a level of from 0.1 to 5wt%, e.g. from 0.5 to 2wt%.

In one embodiment, the particles are treated with an agent selected from stearic acid, trimethoxycaprylylsilane, glycerin, dimethicone, hydrogen dimethicone, simethicone; cetyl phosphate, manganese dioxide, and triethoxycaprylylsilane, and combinations thereof.

In one embodiment, all coatings and treatments for the particles are solely selected from silica, hydrated silica, alumina, aluminium hydroxide, aluminium stearate, stearic acid, trimethoxycaprylylsilane, glycerin, dimethicone, hydrogen dimethicone, simethicone; cetyl phosphate, manganese dioxide, and triethoxycaprylylsilane, and combinations thereof.

In one embodiment, all coatings and treatments for the particles are solely selected from silica, hydrated silica, stearic acid, trimethoxycaprylylsilane, glycerin, dimethicone, hydrogen dimethicone, simethicone; cetyl phosphate, manganese dioxide, and triethoxycaprylylsilane, and combinations thereof.

As the skilled person will appreciate, titanium dioxide is prepared via a process that involves a milling step. A preferred milling step involves the use of a mill selected from fine media mills and sand mills. In such mills fine grinding media, accelerated by means other than gravity, may be used to reduce slurried pigment agglomerates to sub micrometre size.

Particles resulting from the milling step are then coated with silica coating and any optional further coating layer.

The coating of the titanium dioxide may be carried out in a manner similar to that of conventional pigmentary material, as known in the art. It may therefore involve dispersion of the material in water, following which suitable coating reagents, such as sodium silicate, are added. The pH is then adjusted to cause precipitation of the desired hydrated oxide to form a coating onto the surface of the material.

Coatings may generally be achieved by addition of suitable salts to the particulate materials at either an acidic pH (e.g. pH from around 1 to 2) or a basic pH (e.g. pH from around 9.5 to 12), with neutralisation to effect precipitation. The salts may firstly be added followed by subsequently adjustment of the pH: alternatively the pH may be adjusted whilst the salt is being added.

After coating formation, the coated material may be washed and dried before being ground, e.g. in a fluid energy mill or microniser, to separate particles that have been stuck together by the coating and/or drying steps.

At this final milling stage, inorganic or organic surface treatments, e.g. with polyol, amine, alkyl phosphonic acid, silicone or silicone derivatives, may be applied as required.

In one embodiment, the particulate material may be treated to selectively remove particular size fractions. For example, any particles which are 5µm in diameter or greater may be removed; in one embodiment any particles which are 3µm in diameter or greater may be removed. Such particles may be removed by, for example, a centrifugation treatment.

In the cosmetic composition there is from 0.1 to 30wt% of the large crystal titanium dioxide, for example from 0.2 to 30wt%, e.g. from 0.3 to 30wt%. It may be that there is from 0.1 to 25wt% of the large crystal titanium dioxide, for example from 0.2 to 25wt%, e.g. from 0.3 to 25wt%. It may be that there is from 0.1 to 15wt% of the large crystal titanium dioxide, for example from 0.2 to 15wt%, e.g. from 0.3 to 15wt%.

In one embodiment there is from 0.5 to 30wt% of the large crystal titanium dioxide, for example from 0.5 to 25wt%, e.g. from 0.5 to 20wt% or from 0.5 to 15wt%. In one embodiment there is from 1 to 30wt% of the large crystal titanium dioxide, for example from 1 to 25wt%, e.g. from 1 to 20wt% or from 1 to 15wt%. In one embodiment there is from 2 to 30wt% of the large crystal titanium dioxide, for example from 2 to 25wt%, e.g. from 2 to 20wt%.

It may be that there is from 5 to 30wt% of the large crystal titanium dioxide, for example from 5 to 25wt%, e.g. from 5 to 20wt%.

In one embodiment there is from 2 to 15wt% of the large crystal titanium dioxide, for example from 3 to 15wt%, e.g. from 5 to 15wt%. In one embodiment there is from 2 to 10wt% of the large crystal titanium dioxide, for example from 3 to 10wt%, e.g. from 5 to 10wt%.

### Organic cosmetic active ingredient with ligand characteristics

The composition of the invention includes an organic cosmetic active ingredient that has ligand characteristics. It may include only one such organic cosmetic active ingredient, or it may include two or more such organic cosmetic active ingredients.

This organic cosmetic active ingredient is required to be organic in the sense that it must contain a carbon-hydrogen bond.

A 'cosmetic active ingredient' refers to a component that can be used in cosmetic compositions to impart an effect. It may, for example, impart a sun protective effect, such as a UV absorption effect or a UV scattering effect, or it may impart an antioxidant effect, or it may impart a self-tanning effect, or it may impart an emulsifying effect. The effects are not limited to those in this list; the skilled reader will be aware of other active ingredient that can be used in cosmetic compositions and their associated effects.

An ingredient that has 'ligand characteristics' refers to a component that can bond to (or ligate to) another component. Thus it is an organic molecule that has one or more functional group capable of adsorbing (physically or chemically) onto another component.

Specifically, it may be an organic molecule that has one or more functional group capable of adsorbing (physically or chemically) onto titanium dioxide, where the titanium dioxide is in the rutile form and has a geometric weight mean crystal size of from 0.2µm to 0.3µm, and wherein the titanium dioxide particles are provided with an alumina coating. These characteristics are typical for the pigmentary TiO₂ of the type conventionally used in cosmetic compositions. An example of such material is SACHTLEBEN® RDI-S.

In the present invention, the ingredient that has 'ligand characteristics' can therefore adsorb to pigmentary TiO₂ of the type conventionally used in cosmetic compositions and in particular it may be an ingredient that undergoes one or more changes in properties as a result of this adsorption.

These changes in properties include, but are not limited to: change in colour; and/or creation of a coloured species; and/or loss of efficacy for the active ingredient. There may be consequential undesired effects for a composition comprising the active ingredient when this bonding occurs, including adverse changes in organoleptic properties and/or reduction or loss of activity.

Where the active ingredient is an emulsifier, this can lead to destabilisation of emulsion compositions containing the emulsifier. Where the active ingredient is an antioxidant, this can lead to reduction or loss in the antioxidant properties of the compositions containing the antioxidant and/or discoloration. Where the active ingredient is a self-tanning agent, this can lead to reduction or loss in the self-tanning properties of the compositions containing the self-tanning agent and/or discoloration. Where the active ingredient is a sun protective agent, e.g. a UV absorber, this can lead to reduction or loss in the sun protective properties (e.g. UV protective properties) of the compositions containing the sun protective agent and/or discoloration.

As noted above, the adsorption may be chemical or physical. Reference to bonding therefore encompasses metallic, covalent and ionic bonds, as well as dipole-dipole interactions, van der Waals forces, London dispersion forces and hydrogen bonding.

In one embodiment the cosmetic active ingredient that has ligand characteristics is selected from keto-enol UV absorbers, especially UV-A absorbers which are dibenzoylmethane derivatives, such as avobenzone. Dibenzoylmethane derivatives are described in US Patents 4,489,057, 4,387,089 and 4,562,067.

Specific examples of keto-enol UV absorbers include avobenzone, acetylacetone, benzoylacetone, dibenzoylmethane, naphthyl benzoylmethane, and indole benzoylmethane. In one embodiment the cosmetic active ingredient that has ligand characteristics is selected from this list, and combinations thereof.

In one embodiment the cosmetic active ingredient that has ligand characteristics is selected from antioxidants.

The antioxidant may be selected from ascorbic acid and derivatives thereof, including ascorbyl palmitate, ascorbic acid, sodium ascorbate, potassium ascorbate, calcium ascorbate, L-ascorbic acid phosphate ester, magnesium ascorbyl phosphate, sodium ascorbyl phosphate, ascorbyl sulfate, sodium ascorbyl 2 phosphate salt and ascorbyl-2-glucoside, and combinations thereof. In one embodiment the antioxidant is ascorbyl palmitate.

The antioxidant may alternatively or additionally be selected from phenolic acids, including chlorogenic acid, ellagic acid, gallic acid, methyl gallate, ethyl gallate, propyl gallate, butyl gallate and pentyl gallate, and combinations thereof. In one embodiment the antioxidant is propyl gallate.

The antioxidant may alternatively or additionally be selected from tocopherols (vitamin E and derivatives thereof). These include naturally occurring vitamin E, synthetic vitamin E, enantiomerically pure forms of vitamin E (e.g. (+)-alpha-tocopherol), vitamin E derivatives such as acetates, succinates, linoleate, more water-soluble forms of vitamin E such as tocophereth-5, tocophereth-10, tocophereth-12, tocophereth-18, tocophereth-50, D-alpha-tocopherol polyethylene glycol 1000-succinates, and combinations thereof. Specific examples of tocopherol or derivatives thereof include α-tocopherol, β-tocopherol, γ-tocopherol, δ-tocopherol, acetic acid-α-tocopherol, nicotinic acid-α-tocopherol, linoleic acid-α-tocopherol, succinic acid -α-tocopherol, as well as α-tocotrienol, β-tocotrienol, γ-tocotrienol, and δ-tocotrienol. In one embodiment the antioxidant is selected from di-alpha- tocopherol (vitamin E) and di-alpha-tocopheryl acetate.

The antioxidant may alternatively or additionally be selected from vitamin A and derivatives thereof. These include naturally occurring vitamin A, vitamin A-palmitate and vitamin A-acetate, as well as beta-carotene. In one embodiment the antioxidant is vitamin A-palmitate.

The antioxidant may alternatively or additionally be selected from erythorbic acid and derivatives thereof. Examples of erythorbic acid or derivatives thereof include erythorbic acid or derivative thereof, such as erythorbic acid, sodium erythorbate, potassium erythorbate, calcium erythorbate, erythorbic acid phosphate, erythorbic acid sulfate and combinations thereof.

In one embodiment the antioxidant may be selected from ascorbyl palmitate, propyl gallate, di-alpha- tocopherol (vitamin E), di-alpha-tocopheryl acetate, or vitamin A-palmitate, and combinations thereof. In one embodiment the antioxidant may be selected from ascorbyl palmitate and propyl gallate, and combinations thereof.

In one embodiment the cosmetic active ingredient that has ligand characteristics is selected from self-tanning agents.

In one embodiment the self-tanning agent may be selected from dihydroxyacetone, erythrulose, lawsone, tyrosine, jugulone, glyceraldehyde, methyl glyoxal, glycerol aldehyde, alloxan, 2,3-dihydroxysuccindialdehyde, 2,3-dimethoxysuccindialdehyde, 2-amino-3-hydroxy-succindialdehyde, 2-benzylamino-3-hydroxysuccindialdehyde, 6-aldo-D-fructose, hydroxymethylglyoxal, mucondialdehyde, and malealdehyde, and combinations thereof.

In one embodiment the self-tanning agent may be a ketose, e.g. it may be selected from dihydroxyacetone and erythrulose, and combinations thereof.

In one embodiment the cosmetic active ingredient that has ligand characteristics is selected from emulsifiers.

In one embodiment the emulsifier is an acrylic or acrylate emulsifier.

It may be, for example, that the emulsifier is sodium polyacrylate, or an acrylate/C10-30 alkyl acrylate crosspolymer, or a carbomer (non-linear polymer of acrylic acid, optionally crosslinked with polyalkenyl polyethers or divinyl glycol), or combinations thereof.

In one embodiment the cosmetic active ingredient that has ligand characteristics is selected from keto-enol UV absorbers (e.g. avobenzone), antioxidants (e.g. ascorbyl palmitate, propyl gallate, di-alpha- tocopherol, di-alpha-tocopheryl acetate, or vitamin A-palmitate), ketose self-tanning agents (e.g. dihydroxyacetone), and acrylic or acrylate emulsifiers (e.g. sodium polyacrylate), and combinations thereof.

In one embodiment the cosmetic active ingredient that has ligand characteristics is selected from keto-enol UV absorbers (e.g. avobenzone), ascorbic acid and derivatives thereof (e.g. ascorbyl palmitate), phenolic acids (e.g. propyl gallate), ketose self-tanning agents (e.g. dihydroxyacetone), and acrylic or acrylate emulsifiers (e.g. sodium polyacrylate), and combinations thereof.

In one embodiment the cosmetic active ingredient that has ligand characteristics is selected from avobenzone, ascorbyl palmitate and propyl gallate, and combinations thereof.

In the cosmetic composition there is from 0.1 to 20wt% of the cosmetic active ingredient that has ligand characteristics, for example from 0.2 to 20wt%, e.g. from 0.3 to 20wt%. In one embodiment there is from 0.25 to 20wt% of the cosmetic active ingredient that has ligand characteristics, for example from 0.25 to 15wt%, e.g. from 0.25 to 10wt%.

It may be that there is from 0.15 to 10wt% of the cosmetic active ingredient that has ligand characteristics, for example from 0.15 to 8wt%, or from 0.15 to 5wt%, or from 0.15 to 3wt%. It may be that there is from 0.25 to 8wt% of the cosmetic active ingredient that has ligand characteristics, for example from 0.25 to 5wt%, e.g. from 0.25 to 3wt%.

In one embodiment there is from 0.5 to 20wt% of the cosmetic active ingredient that has ligand characteristics, for example from 0.5 to 15wt%, e.g. from 0.5 to 10wt%. It may be that there is from 0.5 to 8wt% of the cosmetic active ingredient that has ligand characteristics, for example from 0.5 to 5wt%, e.g. from 0.5 to 3wt%.

In one embodiment there is from 1 to 20wt% of the cosmetic active ingredient that has ligand characteristics, for example from 1 to 15wt%, e.g. from 1 to 10wt%. It may be that there is from 1 to 8wt% of the cosmetic active ingredient that has ligand characteristics, for example from 1 to 5wt%, e.g. from 1 to 3wt%.

It may be that the large crystal titanium dioxide and the cosmetic active ingredient that has ligand characteristics are used within certain weight ratios. In one embodiment the weight ratio of large crystal titanium dioxide to the cosmetic active ingredient that has ligand characteristics is from about 100:1 to about 1:20, e.g. from about 100:1 to about 1:10 or from about 75:1 to about 1:10, such as from about 50:1 to about 1:5 or from about 50:1 to 1:3.

### Cosmetically acceptable carrier

The composition includes a cosmetically acceptable carrier. It may include a blend or mixture of two or more cosmetically acceptable carriers.

The cosmetically acceptable carrier may be oil or wax based and/or water based.

For example, it may be water based and may comprise de-ionized water, purified water, natural spring water, rose water or the like. In one embodiment de-ionized or purified water is used.

The water based carrier may be 100% water or it may comprise components other than water, including but not limited to water- soluble moisturizing agents, conditioning agents, antimicrobials, humectants (e.g. glycerin) and/or other water- soluble skin care actives.

In another embodiment, the carrier may be oil or wax based. The oil may be natural oil or synthetic oil. The wax is preferably a natural wax.

Combinations of one or more oils and/or one or more waxes may be used.

Liquid oils that can be mentioned include avocado oil, Camellia oil, turtle bean oil, macadamia nut oil, corn oil, mink oil, olive oil, Canoga oil, egg yolk oil, sesame seed oil, Persic oil, wheatgerm oil, Camellia sasanqua oil, castor oil, linseed oil, safflower oil, sunflower oil, grapeseed oil, apricot oil, shea oil, sweet almond oil, cotton oil, evening primrose oil, palm oil, perilla oil, hazelnut oil, soybean oil, peanut oil, tea seed oil, kaya oil, rice bran oil, rapeseed oil, alfalfa oil, Chinese tung tree wood oil, Japanese tung tree wood oil, jojoba oil, germ oil, poppyseed oil, pumpkin oil, blackcurrant oil, millet oil, barley oil, quinoa oil, rye oil, candlenut oil, passionflower oil, musk rose oil, triglycerine, glyceryl trioctanoate, and glyceryl triisopalmitate.

Solid oils/fats that can be mentioned include cocoa butter, coconut butter, horse fat, hardened coconut oil, palm oil, beef tallow, mutton tallow, hardened beef tallow, palm kernel oil, lard, Japan wax kernel oil, hardened oil, Japan wax, shea butter, and hardened castor oil;

Waxes that can be mentioned include beeswax, candelilla wax, carnauba wax, lanolin, lanolin acetate, liquid lanolin, sugar cane wax, fatty acid isopropyl lanolin, hexyl laurate, reduced lanolin, jojoba wax, hard lanolin, polyoxyethylene (hereinafter referred to as POE), lanolin alcohol ether, POE lanolin alcohol acetate, lanolin fatty acid polyethylene glycol, and POE hydrogenated lanolin alcohol ether. In one embodiment the carrier is not lanolin based.

Ester oils that can be mentioned include isopropyl myristate, cetyl octoate, octyldodecil myristate, isopropyl palmitate, butyl stearate, hexyl laurate, myristyl myristate, decyloleate, hexyldecyl dimethyl octoate, cetyl lactate, myristyl lactate, lanolin acetate, isocetyl stearate, isocetyl iso-stearate, 12-hydroxy cholesteryl stearate, di-2-ethylhexylic acid ethyleneglycol, dipentaerythritol fatty acid ester, N-alkylglycol monoisostearate, neopentylglycol dicaprate, diisostearyl malate, glyceryl di-2-heptyl undecanate, tri-methylol propane tri-2-ethylhexyl acid, tri-methylol propane triisostearate, pentaerythritol tetra-2-ethylhexyl acid, glyceryl tri-2-ethylhexanoate, tri-methylol propane triisostearate, cetyl-2-ethylexanoate, 2-ethylhexyl-palmitate, glycerine trimyristate, glyceride tri-2-heptyl undecatoic acid, methyl ester of castor oil fatty acid, oleate oil, acetoglyceride, palmitate-2-heptyl undecyl, diisopropyl adipate, N-lauroyl-L-glutamic acid-2-octyldodecil ester, di-2-heptylundecyl adipate, di-2-ethylhexyl sebacate, myristate-2-hexyldecyl, palmitate-2-hexyldecyl, adipate-2-hexyldecyl, diisopropyl sebacate, and succinate-2-ethylhexyl.

Higher fatty acids that can be mentioned include lauric acid, myristic acid, palmitic acid, stearic acid, behenic acid, oleic acid, 12-hydroxy-stearic acid, undecylenic acid, lanolin fatty acid, isostearic acid, linolic acid, linolenic acid, and eicosapentaenoic acid.

Higher alcohols of straight/branched chain that can be mentioned include lauryl alcohol, cetyl alcohol, stearyl alcohol, behenyl alcohol, myristyl alcohol, oleyl alcohol, cetostearyl alcohol, monostearyl glycerine ether (batyl alcohol), 2-decyltetradecinol, lanolin alcohol, cholesterol, phytosterol, hexyldodecanol, isostearyl alcohol, octyldodecanol.

In one embodiment, the carrier is present in an amount of from 5 to 99.8wt%, such as from 5 to 99.5wt%, or from 10 to 99 wt % or from 10 to 98 wt % or from 15 to 97 wt % or from 15 to 95 wt %. It may be that the carrier is present in an amount of from 20 to 90 wt%, such as from 20 to 85 wt % or from 25 to 80 wt % or from 25 to 75 wt % or from 30 to 70 wt %.

The carrier usefully contains water. Typical water levels in the composition may be from 5% to 99 wt%, e.g. from 10% to 95wt%, or from 15% to 90wt%.

### "Small crystal" TiO₂ material

The composition of the fourth aspect includes from 0.1 to 30wt% of titanium dioxide particulate material, wherein the titanium dioxide is in the rutile form and has a geometric weight mean crystal size of up to 0.2µm, such as 0.15µm or less.

This small crystal titanium dioxide may be ultrafine titanium dioxide or may be pigmentary titanium dioxide, or may be a combination thereof.

It may have a geometric weight mean crystal size of from 0.005 up to 0.2µm, e.g. from 0.010 up to 0.2µm.

It may have a geometric weight mean crystal size of from 0.005 up to 0.15µm, e.g. from 0.010 up to 0.15µm.

It may have a geometric weight mean crystal size of from 0.005 up to 0.1µm, e.g. from 0.010 up to 0.1µm.

This small crystal titanium dioxide may be coated or uncoated.

In one embodiment it has a coating layer and this comprises one or more material selected from inorganic oxides and phosphates. For example, it may comprise one or more inorganic oxide independently selected from an oxide of Ti, Si, Al, Zr, Zn, P, Sn and Ce and/or one or more inorganic phosphate independently selected from a phosphate of Al, Ti, Zr, and Sn.

In one embodiment, it has at least a silica coating.

It may be that the large crystal titanium dioxide and the small crystal titanium dioxide are used within certain weight ratios. In one embodiment the weight ratio of large crystal titanium dioxide to the small crystal titanium dioxide is from about 10:1 to about 1:10, e.g. from about 6:1 to about 1:6, such as from about 3:1 to about 1:3 or from about 2:1 to 1:2.

### Cosmetic composition

The invention relates to cosmetic compositions. Accordingly, all components of the composition must be cosmetically acceptable.

The cosmetic compositions may be, for example, in the form of creams, gels, lotions, oils or pastes, alcoholic and aqueous/alcoholic solutions, emulsions, wax/fat compositions, stick preparations, powders or ointments.

In general, the compositions of the present invention are topical compositions that can be provided in a variety of forms, including but not limited to lotions, milks, mousses, serums, sprays, aerosols, foams, sticks, gels, creams and ointments. In one embodiment, the composition is in the form of a spray or gel and in another embodiment the composition is in the form of a lotion, milk or cream.

The composition may be a water-based composition, oil-based composition, or emulsion composition.

Examples of the water-based composition include skin lotions, beauty essences, water-based gels, and the like, while examples of the oil-based composition include cleansing oil and oil-based gels, and the like. Examples of the emulsion composition include creams, skin milks and sunscreen lotions, and the like. The types of emulsion include oil in water emulsion (o/w), water in oil emulsion (w/o) and multilayer emulsion (e.g. w/o/w, o/w/o).

The final formulations may therefore exist in a wide variety of presentation forms, for example:
- in the form of liquid preparations as a w/o, o/w, o/w/o, w/o/w or PIT emulsion or in the form of a microemulsion,
- in the form of a gel,
- in the form of an oil, a cream, a milk or a lotion,
- in the form of a powder, or a lacquer, or a compressed tablet or stick of make-up,
- in the form of a spray (spray with propellent gas or pump-action spray) or an aerosol,
- in the form of a foam, or
- in the form of a paste.

As water- and oil-containing emulsions (e.g. w/o, o/w, o/w/o and w/o/w emulsions or microemulsions) the cosmetic compositions may contain, for example,
from 1 to 60% by weight, especially from 5 to 50% by weight and preferably from 10 to 35% by weight, based on the total weight of the composition, of at least one oil component,
from 0 to 30% by weight, especially from 1 to 30% by weight and preferably from 4 to 20% by weight, based on the total weight of the composition, of at least one emulsifier,
from 10 to 90% by weight, especially from 30 to 90% by weight, based on the total weight of the composition, of water,
and from 0 to 88.9% by weight, especially from 1 to 50% by weight, of further cosmetically acceptable adjuvants.

In one embodiment, the compositions of the invention are provided as sunscreen agents, including sunblock agents. However, the compositions of the invention are not limited to those compositions applied to the skin primarily as a sunscreen agent. The compositions also include formulations where a sunscreen active agent is an ingredient in another topically applied composition. Non-limiting examples are: make-up (e.g. foundation, eye-shadow, lipstick or lip gloss); lip balms; face creams, lotions, primers and balms; eye creams, gels, concealers and primers; hand creams and lotions; hair dyes and conditioners; or any other cosmetic product where sun protection may be deemed beneficial.

In general, the cosmetic composition may be selected from:
- skin-cleaning preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, especially moisturisers for the face or body, e.g. skin emulsions, multi-emulsions or skin oils; including lip balms; face creams, lotions, and balms; eye creams and gels; hand creams and lotions;
- cosmetic personal care preparations, e.g. facial make-up in the form of foundation and primers, face powder (loose or pressed), eye preparations, e.g. eyeshadow, mascara, eyeliner, eye concealers and primers; lip preparations, e.g. lipsticks, lip gloss, lip contour pencils; nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments, hair-structuring preparations, e.g. hair-waving preparations for permanent waves, hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.

The compositions of this invention are applied topically. In one embodiment they are applied to the skin as a liquid rub-on or as a spray.

In one embodiment, the composition is a sunscreen composition. Sunscreen compositions are typically categorized as either aqueous or non-aqueous. Aqueous sunscreen compositions are typically creams formed as emulsions containing the active UV absorbing compounds and additional ingredients such as waterproofing agents, fragrances, emollients and other skin care ingredients. Non-aqueous sunscreen compositions are those that are typically solvent based compositions that can be formed as gels for topical application or sprayed on, for example from an alcohol based solution of the ingredients.

Sunscreen compositions may be prepared as aqueous volatile solvent-based compositions, meaning non-emulsion compositions containing primarily volatile solvents and up to about 30% by weight water. Thus, the compositions may comprise a single liquid phase that may further comprise dispersed particulates. In certain embodiments, the compositions of the invention contain up to about 25% by weight water or up to about 20% by weight water. In some embodiments of the invention the compositions comprise between about 10% and about 30% by weight water, between about 10% and about 25% water or between about 10% and about 20% water.

Examples of suitable volatile solvents include one or more of alcohols such as methanol, ethanol and isopropanol, volatile hydrocarbons such as isooctane, isododecane, and isohexadecane, aldehydes and volatile silicones also including volatile ketones such as acetone and methyl ethyl ketone. In one embodiment the volatile solvent is chosen from the group consisting of ethanol, methanol, isopropanol and acetone. The sunscreen compositions of the invention containing alcohol based solvent systems are characterized as non-aqueous solutions. However, it may be desirable to have small amount of water in the composition, for example as a processing aid or co-solvent. In certain example embodiments, the water contents of the compositions will be no greater than about 9% water so as to prevent the active to phase separate or precipitate out of solution. The skilled reader will recognise that different actives have different tolerance for water in solution and will adjust the water content accordingly.

Additionally, the solvent can include an oil such as mineral or vegetable oil. The oil may be the only solvent or may be used in varying amounts as a co-solvent or as an emollient.

In certain embodiments, the compositions can be stored in containers under pressure by combination with a propellant. The compositions thus stored can be applied by opening a valve in the container releasing the propellant and the composition, typically in a spray or mist. The propellant used in the composition may be any suitable gas, or combination of gasses, that can be compressed or liquefied within a dispensing spray canister, which expand or volatilize to vapour or gas form upon exposure to ambient temperature and pressure conditions to deliver the composition in an aerosol form. Suitable propellants include hydrocarbons having 1 to 5 carbon atoms, including but not limited to methane, ethane, propane, isopropane, butane, isobutane, butene, pentane, isopentane, neopentane, pentene, hydro fluorocarbons (HFCs), chlorofluorocarbons (CFCs), nitrogen, ethers including dimethyl ether, and any mixtures thereof.

The skilled reader will appreciate that in a closed container such as an aluminium can or glass bottle, propellants such as dimethyl ether condense to the liquid state at ambient temperature. Thus, the composition in the aerosol container is a liquid formulation which can contain dissolved propellant, undissolved liquid propellant and gaseous propellant. All of this is under pressure due to the vapour pressure of the propellant. The propellant can be present in an amount up to 90 wt%, preferably from 2 wt%to 50 wt%, e.g. from 5 wt% to 40 wt%, based on the total weight of the aerosol composition.

### Optional components

The International Cosmetic Ingredient Handbook, 16th Edition, 2016 (Editors: Joanne Nikitakis and Beth Lange, Ph.D; Published by the Personal Care Products Council) describes a wide variety of non-limiting cosmetic and pharmaceutical ingredients commonly used in the skin care industry, which are suitable optional components for use in the compositions of the present invention.

Examples of the functional classes of optional components that may be present include: absorbents, abrasives, anticaking agents, antifoaming agents, antimicrobials, antioxidants, binders, biological additives, buffering agents, bulking agents, chelating agents, chemical additives, colorants and pigments, cosmetic astringents, cosmetic biocides, denaturants, drug astringents, external analgesics, film formers, fragrance components, humectants, opacifying agents, pH adjusters, plant extracts including essential oils, plasticizers, preservatives, propellants, reducing agents, sequestrants, skin bleaching agents, skin-conditioning agents (including emollients and humectants), skin cooling agents, skin protectants, solvents, foam boosters, hydrotropes, solubilizing agents, suspending agents (non-surfactant), sunscreen agents, ultraviolet light absorbers, SPF boosters, waterproofing agents, vitamins, and thickeners/viscosity increasing agents (aqueous and non-aqueous). Any one or more of these types of optional components may be included.

### Additional sunscreens

The compositions, in addition to the titanium dioxide material which has a sunscreen effect, may further include one or more additional sunscreen active agent.

These sunscreen agents may, for example, comprise from 0.1% to 30%, e.g. from 0.5% to 25%, such as from 1% to 20% by weight of the composition. Exact amounts of sunscreen agent will vary depending upon the sunscreen or sunscreens chosen and the desired Sun Protection Factor (SPF) to be achieved.

These sunscreen agents may be selected from: para aminobenzoic acid, avobenzone, cinoxate, dioxybenzone, homosalate, menthyl anthranilate, octyl salicylate, oxybenzone, padimate O, phenylbenzimidazole sulfonic acid, sulisobenzone, trolamine salicylate, diethanolamine methoxycinnamate, digalloy trioleate, ethyl dihydroxypropyl PABA, glyceryl aminobenzoate, lawsone with dihydroxyacetone, red petrolatum; ethylhexyl triazone, dioctyl butamido triazone, benzylidene malonate polysiloxane, terephthalylidene dicamphor sulfonic acid, disodium phenyl dibenzimidazole tetrasulfonate, diethylamino hydroxybenzoyl hexyl benzoate, bis diethylamino hydroxybenzoyl benzoate, bis benzoxazoylphenyl ethylhexylimino triazine, drometrizole trisiloxane, methylene bis-benzotriazolyl tetramethylbutylphenol, and bis-ethylhexyloxyphenol methoxyphenyltriazine, 4-methylbenzylidenecamphor, and isopentyl 4-methoxycinnamate.

In one embodiment the additional sunscreen active agent is organic.

In one embodiment the additional sunscreen active agent is an organic sunblock.

### Humectants, Moisturizers, and Skin Conditioners

The compositions of the present invention can optionally comprise one or more humectant, moisturizing, or skin conditioning materials. A variety of these materials can be employed and each can be present at a level of from 0.1wt% to 20wt%, e.g. from 1wt% to 10wt%, such as from 2wt% to 5wt%.

These materials include guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g. aloe vera gel); polyhydroxy alcohols such as sorbitol glycerol, hexanetriol propylene glycol, butylene glycol, hexylene glycol and the like; polyethylene glycols; sugars and starches; sugar and starch derivatives (e.g. alkoxylated glucose); hyaluronic acid; lactamide monoethanolamine; acetamide monoethanolamine; and mixtures thereof.

Also useful are various C1-C30 monoesters and polyesters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatly acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatly acids, xylitol pentaoleate, sucrose tetraoleate, sucrose pentaoletate, sucrose hexaoleate, sucrose hepatoleate, sucrose octaoleate, and mixtures thereof.

### Emulsifiers

The compositions of the present invention can also comprise one or more emulsifiers. Suitable emulsifiers can include any of a wide variety of non-ionic, cationic, anionic, and zwitterionic emulsifiers.

The emulsifiers can be used individually or as a mixture of two or more. Emulsifiers can suitably comprise from 0.1wt% to 10wt%, e.g. from 0.15wt% to 7wt%, such as from 0.25wt% to 5wt% of the compositions of the present invention.

Suitable emulsifier types include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers, ethoxylated alcohols, alkyl phosphates, polyoxyethylene fatty ether phosphates, fatty acid amides, acyl lactylates, soaps and mixtures thereof.

Suitable emulsifiers can also include, but are not limited to, DEA oleth-3 phosphate, polyethylene glycol 20 sorbitan monolaurate (polysorbate 20), polyethylene glycol 5 soya sterol, steareth-2, steareth-20, steareth-21, ceteareth-20, PPG-2 methyl glucose ether distearate, ceteth-10, polysorbate 80, cetyl phosphate, potassium cetyl phosphate, diethanolamine cetyl phosphate, polysorbate 60, glyceryl stearate, PEG-100 stearate, and mixtures thereof.

### Examples

The invention will be further described with reference to the non-limiting examples.

### Example 1

A series of sunscreen formulations were prepared, which each included rutile titanium dioxide. The titanium dioxide materials varied in terms of their coatings and in terms of their crystal size. The details of how the sunscreens differed are set out in Table 1.

**Table 1**

| **Product** | **Coating on TiO₂** | **Geometric weight mean crystal size of TiO₂ (microns)** |
|---|---|---|
| Reference | 20wt% silica | 0.02 |
| Sunscreen A | Uncoated | 1.00 |
| Sunscreen B | Uncoated | 0.70 |
| Sunscreen C | Uncoated | 0.40 |
| Sunscreen D | 2.5wt% alumina | 1.00 |
| Sunscreen E | 2.5wt% silica and 2.5wt% alumina | 1.00 |
| Sunscreen F | 2.5wt% silica and 2.5wt% alumina | 0.70 |
| Sunscreen G | 2.5wt% silica and 2.5wt% alumina | 0.40 |

Each sunscreen was prepared as follows:
A dispersion of the TiO₂ material was prepared by speed-mixing 5g of the titania into 5g of glycerine over 3 minutes at 2500rpm.

A water phase was then prepared by combining the aqueous components according to Table 2 below and speed-mixing for 1 minute at 1000rpm.

**Table 2**

| **Component** | **Amount (g)** |
|---|---|
| Water | 36.0 |
| Glycerine | 5.0 |
| TiO₂/Glycerine 5g/5g dispersion | 10.0 |
| Preservative ¹ | 1.0 |

| | |
|---|---|
| ¹ Germaben II-E - Available from International Specialty Products | |

An oil phase was prepared by combining the components in Table 3 below:

**Table 3**

| **Component** | **Amount (g)** |
|---|---|
| Polyalkylene glycol emulsion stabilizer ² | 10.8 |
| Hydroxyoctacosanyl hydroxystearate non-bleached wax and consistency modifier ³ | 5.4 |
| Ethylhexyl palmitate | 21.8 |
| Dimethicone | 3.0 |
| Vitamin E acetate | 1.0 |
| Base fluid: blend of volatile polydimethylcyclosiloxane composed of cyclohexasiloxane and cyclopentasiloxane ⁴ | 6.0 |

| | |
|---|---|
| ² Elfacos® ST9 - Available from AkzoNobel ³ Elfacos® C26 - Available from AkzoNobel ⁴ Xiameter® PMX-0345- Available from Dow Corning | |

The oil phase was heated to 80°C and melted. The water phase was heated to 70°C. The heated water phase was slowly added to the melted oil phase, with constant stirring to produce an emulsion. The emulsion was allowed to cool.

For each sunscreen thus formed, the following tests were carried out.

Sun Protection Factor (SPF) and Ultra Violet A Protection Factor (UVAPF) were determined *in vitro* using the COLIPA method ISO 24443:2012 "Determination of sunscreen UVA photoprotection *in vitro".*

The *in vitro* UVA protection factor (UVAPF) is the protection factor of a sun protection product against UVA radiation, which can be derived mathematically with in vitro spectral modelling.

The SPF *in vitro* is the protection factor of a sun protection product against erythema-inducing radiation calculated with spectral modelling.

The tests were carried out on roughened poly(methyl methacrylate) plaques over the wavelength range 280-400nm using a Labsphere UV-2000S spectrometer (UV transmittance analyser).

HELIOPLATE HD 6 (50 mm x 50 mm) PMMA plates having about 6 microns roughness, available from HelioScreen Laboratories, can be used in this regard.

Transparency was also measured, as ΔL* over a black background.

In this test, the sunscreen was applied by a gloved finger on an area of a matt black card at a spreading rate of 2mg/cm². The sunscreen was left overnight and measurements were taken the next morning. Results (L*) are measured on a spectrometer (X-Rite CE7000A) and the corresponding results from an untreated matt black card are subtracted.

The resulting L* difference (ΔL*) is a measure of transparency.

The results are shown in Table 4 below.

**Table 4**

| **Product** | **SPF** | **UVAPF** | **Transparency ΔL*** |
|---|---|---|---|
| Reference | 8 | 5 | 24 |
| Sunscreen A | 1 | 1 | 22 |
| Sunscreen B | 2 | 2 | 26 |
| Sunscreen C | 2 | 2 | 34 |
| Sunscreen D | 2 | 2 | 45 |
| Sunscreen E | 1 | 1 | 30 |
| Sunscreen F | 1 | 1 | 37 |
| Sunscreen G | 1 | 1 | 46 |

It can be seen that all of the formulations have both UV-A and UV-B protective ability.

The compositions where the titania crystal size is in the range of 0.4 to 0.7 microns are more opaque than the compositions where the titania crystal size is in the range of 0.7 to 1.0 microns. The presence of an alumina coating also increases the opacity.

It will be appreciated that for some cosmetic formulations, opacity is desirable, e.g. when the formulation will cover undesired features on the skin (such as pigmentation or discoloration, marks or scars, and blemishes).

### Example 2

A further series of sunscreens were prepared where each sunscreen formulation included one of avobenzone, ascorbyl palmitate and propyl gallate. These are cosmetic components with a known susceptibility to discoloration and can be described as organic cosmetic active ingredients that have ligand characteristics.

Each sunscreen also included rutile titanium dioxide. The titanium dioxide materials varied in terms of their coatings and in terms of their crystal size. The details of how the sunscreens differed are as set out in Table 1 above.

### Preparation of sunscreens including avobenzone:

A 40wt% dispersion of the TiO₂ component was prepared in caprylic/capric triglyceride oil (Miglyol® 812 Neutral, available from Cremer Oleo GmbH).

A 17wt% dispersion of avobenzone (Parsol® 1789, available from DSM Nutritional Products Europe Ltd) was prepared by dispersing the avobenzone in benzoate ester solvent (Finsolv® TPP, available from Innospec Performance Chemicals, which comprises C12-C15 alkyl benzoate and dipropylene glycol dibenzoate).

The avobenzone dispersion was added to the TiO₂ dispersion to give a sunscreen formulation with a 3% loading of avobenzone with respect to TiO₂.

### Preparation of sunscreens including ascorbyl palmitate:

The TiO₂ component and ascorbyl palmitate were dispersed in caprylic/capric triglyceride oil (Miglyol® 812 Neutral, available from Cremer Oleo GmbH).

The relative amounts were: 10wt% TiO₂, 1wt% ascorbyl palmitate and 89wt% caprylic capric triglyceride.

This provided a sunscreen formulation with a 10% loading of ascorbyl palmitate with respect to TiO₂.

### Preparation of sunscreens including propyl gallate:

The TiO₂ component and propyl gallate were dispersed in in benzoate ester solvent (Finsolv® TPP, available from Innospec Performance Chemicals, which comprises C12-C15 alkyl benzoate and dipropylene glycol dibenzoate).

The relative amounts were: 1g TiO₂, 0.025g propyl gallate and 4ml of Finnsolv TPP.

This provided a sunscreen formulation with a 2.5% loading of propyl gallate with respect to TiO₂.

### Testing:

The extent of discoloration for each sunscreen formulation (ΔE*) was measured after 7 days in dark storage at room temperature.

In this test, the sunscreen was applied at a rate of 2mg/cm² and L8,a*,b* values were measured over a quartz plate immediately. The preparation was applied to another quartz plate after 7 days of storage and again the colour was measured. The colour differences were then calculated. The colour measurements were made on a Konica Minolta CR-410 Colorimeter.

ΔE* is the measured distance in perceptual color space. Differences below 0.2 are considered negligible.

The results are shown in Table 5 below.

**Table 5**

| **Product** | **Avobenzone ΔE*** | **Ascorbyl Palmitate ΔE*** | **Propyl Gallate ΔE*** |
|---|---|---|---|
| Reference | 30.0 | 19.0 | 29.0 |
| Sunscreen A | 3.5 | 5.4 | 10.8 |
| Sunscreen B | 3.6 | 6.1 | 11.7 |
| Sunscreen C | 4.4 | 7.8 | 12.6 |
| Sunscreen D | 4.0 | 4.1 | 12.0 |
| Sunscreen E | 2.3 | 1.0 | 1.3 |
| Sunscreen F | 2.2 | 0.4 | 1.5 |
| Sunscreen G | 2.7 | 0.4 | 2.3 |

It can be seen that all the sunscreen formulations containing titania with crystal sizes greater than 0.35 microns have significantly reduced discoloration as compared to the reference formulations containing smaller crystal size titania. This is surprising and would have not been predicted. This effect was seen for all three of the tested cosmetic components with a known susceptibility to discoloration.

The best results for reduction in discoloration were obtained for the three formulations E, F and G where the titania was silica coated as well as having geometric weight mean crystal size greater than 0.35 microns. For these products, the ΔE* value was reduced by more than 90%, and in several cases more than 95%, as compared to the reference.

It is highly significant that these specific forms of titania have been identified that can be used together with cosmetic components previously known for discolouring in the presence of minerals such as TiO₂, but without causing significant discolouration.

### Example 3

A series of sunscreens were prepared, each including one or more type of rutile titanium dioxide.

The types of rutile titanium dioxide used were as set out in Table 6 below.

Each TiO₂ product was first prepared as a dispersion by speed-mixing 5g of the TiO₂ into a vehicle, over 3 minutes at 2500rpm.

In each case a suitable vehicle was chosen and different ratios of pigment to vehicle were needed to achieve a viscosity suitable for subsequent processing. In general, the smaller the size of the TiO₂, the higher the surface area and the greater the demand for vehicle to disperse the titania.

**Table 6**

| **Product** | **Geometric weight mean crystal size of TiO₂ (microns)** | **Surface treatment** | **Dispersion TiO₂ plus vehicle** |
|---|---|---|---|
| Large crystal TiO₂: Titania F | 0.70 | 2.5wt% silica and 2.5wt% alumina | 5g Titania F +5g glycerine |
| Ultrafine rutile TiO₂: UV-Titan® M195 (Venator Corp) | 0.015 | 15wt% SiO₂ ⁵ | 5g M195 + 7.5g isononyl isononaoate/ polyhydroxystearic acid ⁶ |
| Pigmentary rutile TiO₂: Sachtleben® RC402 (Venator Corp) | 0.19 | 0.5wt% SiO₂, 1.2wt% Al₂O₃, and 0.64wt% glycerine | 5g RC402 + 2.1g sorbitan isostearate ⁷ |

| | | | |
|---|---|---|---|
| ⁵Includes SiO₂ provided as a component of hydrogen dimethicone ⁶ Dispersun ® DSP-OL300 - Available from Innospec ⁷ Crill6 - Available from Croda | | | |

These dispersions were incorporated or combined into the oil or water phases of sunscreen formulations, according to Table 7 below.

**Table 7**

| | **Formulation** | | | | |
|---|---|---|---|---|---|
| | **(i)** | **(ii)** | **(iii)** | **(iv)** | **(v)** |
| **Oil Phase** | | | | | |
| Emulsifier: cetyl dimethicone copolyol, polyglyceryl-4-isostearate, hexyl laurate⁸ | 7 | 7 | 7 | 5 | 5 |
| Carrier/base fluid: cyclotetrasiloxane, cyclopentasiloxane ⁹ | 5 | 5 | 5 | 5 | 5 |
| Silicone wax / consistency agent: cetyl dimethicone ¹⁰ | 1 | 1 | 1 | 1 | 1 |
| Octyl stearate | 2 | 2 | 2 | 2 | 2 |
| Water-in-oil emulsifier ¹¹ | 1 | 1 | 1 | 1 | 1 |
| Beeswax | 0.6 | 0.6 | 0.6 | 0.6 | 0.6 |
| Polydecene | 4 | 4 | 4 | 0 | 0 |
| TiO₂: UV-Titan® M195 dispersion | 0 | 12.5 | 0 | 12.5 | 12.5 |
| TiO₂: Sachtleben® RC402 dispersion | 0 | 0 | 7.1 | 0 | 7.1 |
| | | | | | |

| **Oil Phase** | | | | | |
|---|---|---|---|---|---|
| Water (deionised) | 68.8 | 66.3 | 72.0 | 62.3 | 55.2 |
| Sodium chloride | 0.6 | 0.6 | 0.6 | 0.6 | 0.6 |
| TiO₂: Titania F dispersion | 10 | 0 | 0 | 10 | 10 |
| | | | | | |
| Total (g) | 100 | 100 | 100 | 100 | 100 |

| | | | | | |
|---|---|---|---|---|---|
| ⁸ ABIL® WE 09 - Available from Evonik Goldschmidt ⁹Xiameter™ PMX-0344 - Available from Dow Corning ¹⁰ Abil Wax 9801 - Available from Evonik Goldschmidt ¹¹ Olivem® 900 - Available from Hallstar | | | | | |

In each case the oil phase was heated to 80°C and melted. The water phase was heated to 70°C. The heated water phase was slowly added to the melted oil phase, with constant stirring to produce an emulsion. The emulsion was allowed to cool.

For each sunscreen thus formed, the Sun Protection Factor (SPF) was determined using the Colipa method, on roughened poly(methyl methacrylate) plaques over the wavelength range 280-400nm using a Labsphere UV-2000S spectrometer (UV transmittance analyser), as described in Example 1.

The results are shown in **Figure 1****.**

Surprisingly, the formulations containing combinations of titania materials - i.e. formulations (iv) and (v) - had superior SPF values as compared to what would have been expected from the SPF values for the formulations containing the titania materials individually. It would have been expected that there would be a simple additive effect, but the values observed are significantly higher than additive.

Thus there is an unexpected synergy in the SPF when the large crystal titania is used with one or both of the ultrafine and pigmentary titania.

### Example 4

6kg of rutile TiO₂ with a mean crystal size of 0.40 microns was split into three portions. Portion (i) was set aside, while portions (ii) and (iii) were each dispersed with 8g of monoisopropanolamine to give an aqueous suspension at 300g/l.

The dispersions were each sand milled to a particle size of 0.44 microns and coated with 1.9wt% dense silica by adjustment of the pH to 8.5 over 60 minutes. The slurries were separately filtered washed and dried.

Portion (iii) was then further treated by addition of 1.5% polydimethylsiloxane.

All three portions were then micronized separately, at a steam to pigment ratio of 2:1.

These three TiO₂ products were then tested against two commercial TiO₂ products.

Each TiO₂ product was first prepared as a dispersion by speed-mixing 3g into a vehicle, over 3 minutes at 2500rpm.

In each case a suitable vehicle was chosen and different ratios of pigment to vehicle were needed to achieve a viscosity suitable for subsequent processing. In general, the smaller the size of the TiO₂, the higher the surface area and the greater the demand for vehicle to disperse the titania.

The types of rutile titanium dioxide used were as set out in Table 8 below.

**Table 8**

| **Product** | **Geometric weight mean crystal size of TiO₂ (microns)** | **Surface treatment** | **Dispersion TiO₂ plus vehicle** |
|---|---|---|---|
| Portion (i) | 0.40 | None | 3g portion (i) + 3g glycerine |
| Portion (ii) | 0.40 | 1.9% SiO₂ | 3g portion (ii) + 3g glycerine |
| Portion (iii) | 0.40 | 1.9% SiO₂ + 1.5% PDMS | 3g portion (iii) + 1.3g sorbitan isostearate ¹² |
| Ultrafine TiO₂: UV-Titan® M195 (Venator Corp) | 0.015 | 15% SiO₂ ¹³ | 3g M195 + 4.5g isononyl isononaoate/ polyhydroxystearic acid ¹⁴ |
| Pigmentary anatase TiO₂: Hombitan® AC360 (Venator Corp) | 0.19 | 1.2% Al₂O₃ + 0.5% SiO₂ (present as siloxane) | 3g AC360 + 1.3g sorbitan isostearate ¹² |

| | | | |
|---|---|---|---|
| ¹² Crill6 - Available from Croda ¹³ Includes SiO₂ provided as a component of hydrogen dimethicone ¹⁴ Dispersun ® DSP-OL300 - Available from Innospec | | | |

These dispersions were incorporated or combined into the oil or water phases of sunscreen formulations, according to Table 9 below.

**Table 9**

| | **Formulation** | | | | | | | | | | |
|---|---|---|---|---|---|---|---|---|---|---|---|
| | **Control A** | **1** | **2** | **3** | **4** | **5** | **6** | **7** | **8** | **9** | **Control B** |
| **Oil Phase** | Amount (g) | | | | | | | | | | |
| Emulsifier: cetyl dimethicone copolyol, polyglyceryl-4-isostearate, hexyl laurate¹⁵ | 6.3 | 6.0 | 6.0 | 5.0 | 6.0 | 6.0 | 5.0 | 5.0 | 5.0 | 5.0 | 7.0 |
| Carrier/base fluid: cyclopentasiloxane, cyclohexasiloxane ¹⁶ | 6.0 | 6.0 | 6.0 | 5.0 | 6.0 | 6.0 | 5.0 | 5.0 | 5.0 | 5.0 | 5.0 |
| Silicone wax / consistency agent: cetyl dimethicone ¹⁷ | 1.0 | 1.0 | 1.0 | 1.0 | 1.0 | 1.0 | 1.0 | 1.0 | 1.0 | 1.0 | 1.0 |
| Octyl stearate | 9.0 | 9.0 | 9.0 | 2.0 | 2.0 | 2.0 | 2.0 | 2.0 | 2.0 | 2.0 | 2.0 |
| Water-in-oil emulsifier ¹⁸ | 1.7 | 1.7 | 1.7 | 1.0 | 1.7 | 1.7 | 1.0 | 1.0 | 1.0 | 1.0 | 1.0 |
| Beeswax | 1.6 | 1.6 | 1.6 | 0.6 | 1.6 | 1.6 | 0.6 | 0.6 | 0.6 | 0.6 | 0.6 |
| Polydecene | 4.0 | 4.7 | 4.7 | 11.1 | 4.3 | 4.3 | 3.6 | 3.6 | 3.6 | 0 | 4.0 |
| TiO₂: UV-Titan® M195 dispersion | 0 | 0 | 0 | 0 | 7.5 | 7.5 | 7.5 | 7.5 | 7.5 | 7.5 | 12.5 |
| TiO₂: Hombitan® AC360 dispersion | 0 | 0 | 0 | 0 | 0 | 0 | 0 | 4.3 | 4.3 | 4.3 | 0 |
| TiO₂: Portion (iii) dispersion | 0 | 0 | 0 | 4.3 | 0 | 0 | 4.3 | 0 | 0 | 4.3 | 0 |
| | | | | | | | | | | | |

| **Water Phase** | Amount (g) | | | | | | | | | | |
|---|---|---|---|---|---|---|---|---|---|---|---|
| Water (deionised) | 69.9 | 63.4 | 63.4 | 69.4 | 63.3 | 63.3 | 69.4 | 63.4 | 63.4 | 68.7 | 66.3 |
| Sodium chloride | 0.6 | 0.6 | 0.6 | 0.6 | 0.6 | 0.6 | 0.6 | 0.6 | 0.6 | 0.6 | 0.6 |
| TiO₂: Portion (i) dispersion | 0 | 6.0 | 0 | 0 | 6.0 | 0 | 0 | 6.0 | 0 | 0 | 0 |
| TiO₂: Portion (ii) dispersion | 0 | 0 | 6.0 | 0 | 0 | 6.0 | 0 | 0 | 6.0 | 0 | 0 |
| | | | | | | | | | | | |
| Total (g) | 100 | 100 | 100 | 100 | 100 | 100 | 100 | 100 | 100 | 100 | 100 |

| | | | | | | | | | | | |
|---|---|---|---|---|---|---|---|---|---|---|---|
| ¹⁵ ABIL® WE 09 - Available from Evonik Goldschmidt ¹⁶ Xiameter™ PMX-0345 - Available from Dow Corning ¹⁷ Abil Wax 9840 - Available from Evonik Goldschmidt ¹⁸ Olivem® 900 - Available from Hallstar | | | | | | | | | | | |

In each case the oil phase was heated to 80°C and melted. The water phase was heated to 70°C. The heated water phase was slowly added to the melted oil phase, with constant stirring to produce an emulsion. The emulsion was allowed to cool.

For each sunscreen thus formed, the Sun Protection Factor (SPF) was determined, on roughened poly(methyl methacrylate) plaques over the wavelength range 280-400nm using a Labsphere UV-2000S spectrometer (UV transmittance analyser), as described in Example 1.

The results are shown in Table 10.

**Table 10**

| | | Coating on large crystal TiO₂ | | |
|---|---|---|---|---|
| | | No coating | 1.9% SiO₂ | 1.9%SiO₂ + 1.5%PDMS |
| | **SPF value** | | | |
| Control A: No TiO₂ | 1.0 | | | |
| Formulations 1-3: 3wt% large crystal TiO₂ alone | | 1.5 | 1.6 | 1.9 |
| Formulations 4-6: 3wt% large crystal TiO₂ plus 3wt% M195 | | 17.1 | 15.5 | 13.6 |
| Formulations 7-9: 3wt% large crystal TiO₂ 3% plus 3wt% M195 and 3% AC360 | | 23.5 | 25.9 | 18.9 |
| Control B: 5wt% M195 only | 12.7 | | | |

It can be seen that the inclusion of the large crystal TiO₂ leads to an improved SPF.

Significantly, the formulations containing combinations of large crystal TiO₂ together with additional titania materials - i.e. formulations 4-9 - had superior SPF values as compared to what would have been expected from the SPF values for the formulations containing the titania materials individually. Thus there is an unexpected synergistic effect.

Note that the Control Formulation B (M195 alone) has 5wt% loading compared to only 3wt% in the combined formulations. Its SPF value at 3wt% loading would be less.

### Example 5

A sample of the Portion (iii) TiO₂ according to Example 4 was provided, together with two commercial TiO₂ products.

Details are set out in Table 11.

**Table 11**

| **Product** | **Geometric weight mean crystal size of TiO₂ (microns)** | **Surface treatment** |
|---|---|---|
| Large crystal TiO₂: Portion (iii) | 0.40 | 1.9% SiO₂ + 1.5% PDMS |
| Ultrafine TiO₂: UV-Titan® M170 (Venator Corp) | 0.015 | 7% Al₂O₃ + 7% SiO₂ ¹⁹ |
| Ultrafine TiO₂: UV-Titan® M195 (Venator Corp) | 0.015 | 15% SiO₂ ¹⁹ |

| | | |
|---|---|---|
| ¹⁹ Includes SiO₂ provided as a component of hydrogen dimethicone | | |

A number of sunscreen formulations were prepared, each including one or more of these TiO₂ products in combination with avobenzone.

The avobenzone was provided in the form of Parsol® 1789, available from DSM Nutritional Products Europe Ltd, as a 33wt% solution in benzoate ester solvent (Finsolv® TPP, available from Innospec Performance Chemicals).

The TiO₂ products were provided in particulate form.

The oil and water components of these formulations were as shown in Table 12 below.

**Table 12**

| | **Control** | **A** | **B** | **C** | **D** | **E** |
|---|---|---|---|---|---|---|
| **Oil Phase** | Amount (g) | | | | | |
| Dimethicone | 3.6 | 3.6 | 3.6 | 3.6 | 3.6 | 3.6 |
| Polyalkylene glycol emulsion stabilizer ²⁰ | 13.0 | 13.0 | 13.0 | 13.0 | 13.0 | 13.0 |
| Hydroxyoctacosanyl hydroxystearate non-bleached wax and consistency modifier ²¹ | 6.25 | 6.25 | 6.25 | 6.25 | 6.25 | 6.25 |
| Ethyl hexyl palmitate | 26.2 | 22.6 | 22.6 | 22.6 | 19.0 | 19.0 |
| Tocopheryl acetate | 1.2 | 1.2 | 1.2 | 1.2 | 1.2 | 1.2 |
| A vobenzone | 7.2 | 7.2 | 7.2 | 7.2 | 7.2 | 7.2 |
| Carrier/base fluid: cyclopentasiloxane, cyclohexasiloxane ²² | 7.2 | 7.2 | 7.2 | 7.2 | 7.2 | 7.2 |
| TiO₂: Portion (iii) | 0 | 3.6 | 0 | 0 | 3.6 | 3.6 |
| TiO₂: UV-Titan ® M170 | 0 | 0 | 0 | 3.6 | 0 | 3.6 |
| TiO₂: UV-Titan ® M195 | 0 | 0 | 3.6 | 0 | 3.6 | 0 |
| | | | | | | |

| **Water Phase** | Amount (g) | | | | | |
|---|---|---|---|---|---|---|
| Water | 43.2 | 43.2 | 43.2 | 43.2 | 43.2 | 43.2 |
| Glycerine | 6.0 | 6.0 | 6.0 | 6.0 | 6.0 | 6.0 |
| | | | | | | |
| Total (g) | 114.1 | 114.1 | 114.1 | 114.1 | 114.1 | 114.1 |

| | | | | | | |
|---|---|---|---|---|---|---|
| ²⁰ Elfacos® ST9 - Available from AkzoNobel ²¹ Elfacos® C26 - Available from AkzoNobel ²² Xiameter® PMX-0345- Available from Dow Corning | | | | | | |

The oil phase components except the carrier/base fluid Xiameter® PMX-0345 and the titania were weighed into a 150ml glass jar and heated to 60 - 80°C, with preheating using a heating plate and under moderate stirring.

During all following steps the temperature was controlled to remain above 60°C using the heating plate.

After all components had melted, the carrier/base fluid Xiameter® PMX-0345 was added with stirring. A mixture was prepared by speed-mixing for 1 minute at 2500 rpm.

The titania was then added into the oil phase with speed-mixing for approximately 1 minute at 2500 rpm.

The water phase components were weighed into a 100ml beaker, mixed with a glass bar and heated to approximately 60-80°C using a heating plate.

The heated water phase was slowly added to the melted oil phase, with constant stirring to produce an emulsion. The emulsion was allowed to cool. During cooling gentle stirring with a propeller mixer was carried out, to prevent separating and skinning.

The emulsion was then homogenized for 3 minutes using an IKA Ultra Turrax T25 at 13400 min-1.

The formulations were tested to determine SPF values and the UVA/UVB ratio. These values were determined using the Colipa ISO 24443:2012 method, on roughened poly(methyl methacrylate) plaques over the wavelength range 280-400nm using a Labsphere UV-2000S spectrometer (UV transmittance analyser), as described in Example 1.

The results are shown in Table 13.

**Table 13**

| | **Control** | **A** | **B** | **C** | **D** | **E** |
|---|---|---|---|---|---|---|
| SPF | 3 | 3.7 | 8.9 | 9.2 | 11.9 | 11.7 |
| UVA/UVB ratio | 1.68 | 1.54 | 0.95 | 0.95 | 0.95 | 0.94 |

It can be seen that all the formulations including avobenzone plus TiO₂ had an improved SPF as compared to avobenzone alone.

Further, the formulations including avobenzone plus TiO₂ had a UVA/UVB ratio closer to 1 as compared to avobenzone alone, showing a balance of protection against both UVA and UVB radiation.

The extent of discoloration for each sunscreen formulation (ΔE*) was measured.

In this regard, colours (L*,a*,b*) were measured on each sunscreen formulation under D65 illumination on a quartz plate using a Konica Minolta CR-410 Colorimeter. Measurements were taken (1) immediately and (2) after storage at room temperature for 4 weeks in opaque plastic containers.

Discolouration values were calculated by simple subtraction of the two sets of raw colour values.

ΔE* is the measured distance in perceptual color space. Differences below 0.2 are considered negligible.

The results are shown in Table 14 below

**Table 14**

| | **ΔL*(D65)** | **Δa*(D65)** | **Δb*(D65)** | **ΔE*ab(D65)** |
|---|---|---|---|---|
| **Control** | -0.47 | -0.04 | 0.05 | 0.48 |
| **A** | -1.25 | -0.12 | -0.09 | 1.26 |
| **B** | -0.25 | -0.59 | 1.26 | 1.41 |
| **C** | -0.33 | -1.14 | 2.2 | 2.5 |
| **D** | -0.89 | -0.23 | 0.64 | 1.12 |
| **E** | 0.91 | -0.36 | 1.5 | 1.8 |

It can be seen that all of the formulations that include TiO₂ together with avobenzone (A to E) exhibit yellowing over time.

However, unexpectedly, the extent of yellowing is smaller when the TiO₂ is large crystal TiO₂ (A) rather than ultrafine TiO₂ (B and C).

It was also surprising that the presence of the large crystal TiO₂ in combination with ultrafine TiO₂ (D and E) led to a reduced yellowing effect as compared to the equivalent formulations which had ultrafine TiO₂ as the only TiO₂ material (B and C). Therefore these formulations have the benefit of a higher SPF, a balanced UVA/UVB ratio, yet without the extent of discoloration problems that would have been predicted.

### Conclusion

Discoloration of cosmetic formulations is problematic from an aesthetic perspective, but also because it can be indicative of a loss of efficacy for active agents.

The present examples show that large crystal TiO₂, especially when coated with silica, leads to a reduced discolouration effect for avobenzone, ascorbyl palmitate and propyl gallate, all of which are cosmetic components with a known susceptibility to discoloration.

Further, this large crystal TiO₂ does have a protective effect against UV rays, meaning it provides an SPF contribution. Its effect is also a balancing one with avobenzone, permitting a UVA/UVB ratio close to 1.

Large crystal TiO₂ is already known to have an IR protective effect (see, e.g., WO2009/136141 and WO2016/128723), especially in the NIR range. Therefore this material beneficially provides broad-spectrum solar protection.

Furthermore, this large crystal TiO₂ has a synergistic effect when used in combination with small crystal TiO₂ (ultrafine or pigmentary) in that the SPF values achieved from the combination were significantly greater than those that would be expected from a purely additive effect for the individual titania materials. Therefore by using this large crystal TiO₂ in combination with small crystal TiO₂ particularly useful sunscreen formulations can be obtained with high SPF values. The ratio of the large crystal TiO₂ to small crystal TiO₂ can be varied to achieve desired organoleptic properties.

## Claims

1. A cosmetic composition that comprises:
- from 0.1 to 20wt% of an organic cosmetic active ingredient that has ligand characteristics;
- from 0.1 to 30wt% of a first titanium dioxide particulate material, wherein the titanium dioxide is in the rutile form and has a geometric weight mean crystal size of from 0.35µm to 5µm, and wherein the titanium dioxide particles are provided with a silica coating; and
- a cosmetically acceptable carrier.

2. The use of titanium dioxide particulate material in a cosmetic composition to prevent or reduce the discoloration and/or loss of efficacy for an organic cosmetic active ingredient that has ligand characteristics, wherein the titanium dioxide particulate material is as defined in claim 1.

3. The use of the cosmetic composition as defined in claim 1 in a method of preventing or reducing damage to human skin from the harmful effects of solar radiation.

4. A cosmetic composition that comprises:
- from 0.1 to 30wt% of a first titanium dioxide particulate material, wherein the titanium dioxide particulate material is as defined in claim 1;
- from 0.1 to 30wt% of a second titanium dioxide particulate material, wherein the titanium dioxide is in the rutile form and has a geometric weight mean crystal size of up to 0.2µm and
- a cosmetically acceptable carrier.

5. The cosmetic composition according to claim 4, wherein the composition further comprises:
- from 0.1 to 20wt% of an organic cosmetic active ingredient that has ligand characteristics.

6. The invention according to any one of the preceding claims, wherein the first titanium dioxide is has a geometric weight mean crystal size of from 0.35µm to 1.5µm.

7. The invention according to any one of the preceding claims, wherein the first titanium dioxide has an amount of silica coating from 0.1 to 10% w/w, when considering the total weight of the silica with respect to the total weight of the particulate titanium dioxide.

8. The invention according to any one of the preceding claims, wherein the first titanium dioxide does not have an alumina coating and does not contain alumina.

9. The invention according to any one of the preceding claims, wherein the first titanium dioxide has been treated with a silicone.

10. The invention according to any one of the preceding claims, wherein the organic cosmetic active ingredient that has ligand characteristics is selected from UV absorbers, antioxidants, self-tanning agents, and emulsifiers, and combinations thereof.

11. The invention according to claim 10, wherein the organic cosmetic active ingredient that has ligand characteristics is selected from keto-enol UV absorbers, ascorbic acid and derivatives thereof, phenolic acids, vitamin E and derivatives thereof, vitamin A and derivatives thereof, erythorbic acid and derivatives thereof, ketose self-tanning agents, and acrylic or acrylate emulsifiers, and combinations thereof.

12. The invention according to claim 11, wherein the organic cosmetic active ingredient that has ligand characteristics is selected from avobenzone, ascorbyl palmitate and propyl gallate, and combinations thereof.

13. The invention according to any one of the preceding claims, wherein the organic cosmetic active ingredient that has ligand characteristics is present in the cosmetic composition in an amount of from 0.5 to 15wt%.

14. The invention according to any one of the preceding claims, wherein the cosmetic composition is in the form of:
• a liquid preparation as an emulsion or microemulsion,
• a gel,
• an oil, a cream, a milk or a lotion,
• a powder, or a lacquer, or a compressed tablet or stick of make-up,
• a spray or an aerosol,
• a foam, or
• a paste.

15. The invention according to any one of the preceding claims, wherein the cosmetic composition is a sunscreen composition.

16. The invention according to claim 15, wherein the composition further comprises one or more additional sunscreen active agent.
